# EUROPEAN PATENT SPECIFICATION

(11) **EP 0 792 257 B1**
(45) Date of publication and mention of the grant of the patent: **09.04.2003**
(21) Application number: 95942844.2
(22) Date of filing: 17.11.1995
(51) Int. Cl.: C07C 50/18, C07C 50/20, C07C 50/22, C07C 50/24, C07C 50/26, C07C 50/34, C07C 50/36, C07C 229/34, C07D 221/02, C07D 221/04, C07C 309/44, C09B 3/12, C09B 3/50, C09B 3/60, C09B 5/26, C09B 5/62, C09B 69/10

(54) **MATERIAL FOR DICHROIC LIGHT POLARIZERS**
MATERIALIEN FÜR POLARISIERER FÜR DICHROIDES LICHT
MATERIAUX POUR DES POLARISEURS DE LUMIERE DICHROIQUES

(30) Priority: 18.11.1994 RU 94041721; 05.06.1995 RU 95109284; 31.07.1995 RU 95113563; 06.10.1995 RU 95117377; 06.10.1995 RU 95117403
(43) Date of publication of application: 03.09.1997
(60) Divisional application: 01119900.7
(73) Proprietor: Optiva, Inc., South San Francisco CA 94080 (US)
(72) Inventor: KHAN, Ir Gvon, Dolgoprudniy, 141700 (RU); BOBROV, Yuri A., Moscow, 103575 (RU); IGNATOV, Leonid Y., Moscow, 127635 (RU); SHISHKINA, Elena Y., Moscow, 127412 (RU); LAZAREV, Pavel, I., Menlo Park, CA 94025 (US); KURBATOV, Alexey V., Moscow, 129281 (RU)
(74) Representative: Ablewhite, Alan James
(86) International application number: US9514413
(87) International publication number: WO96016015

(56) References cited:
- EP-A- 0 557 121
- EP-B- 0 049 873
- GB-A- 991 976
- US-A- 4 133 775
- US-A- 4 440 541

## Description

### Field of the Invention

The present invention relates to colored and neutral dichroic light polarizers (DLPs) based on organic dyestuffs and to processes of preparation thereof.

### Description of the Related Art

Dichroic light polarizers (DLPs) can be based on the organic dyes having sulfoacids of azo or polycyclic compounds or mixtures thereof. These dyes are capable of forming lyotropic liquid crystal phases that form stable lyotropic liquid crystals and liquid crystal compositions. (See for example PCT/US94/05493, published 08.12.94.)

### SUMMARY OF THE INVENTION

The present invention provides dyes of formula I, III, IV, V, VI, VII, VIII, IX, and X, described in detail below. In the dichroic light polarizers of this invention, the dye molecules are aggregated into particles oriented in a predetermined direction on a surface of a substrate to enable the dye to polarize light transmitted through the dye.

### DETAILED DESCRIPTION OF THE INVENTION

The present invention provides a liquid crystal material that can be used to produce yellow, red, green, and grey dichroic light polarizers having high polarization characteristics (e.g., with K_{d} values not less than 15). The liquid crystal materials of the present invention contain an organic dye represented by any of formulas I, III, IV, V, VI, VII, VIII, IX and X, below or mixtures thereof that are capable of forming the liquid crystal phase.

The materials also facilitate preparation of polarizing coatings with high polarizing characteristics using high velocity application methods. This is accomplished by using a dye of this invention, which dyes are able to form stable lyotropic liquid crystalline phases.

### Dyes I-XXXIV

The dyes capable of forming lyotropic liquid crystals of this invention are described below. As used herein dyes of formulas I, III, IV, V, VI, VII, VIII, IX and X are as described below. Abbreviations used in the formulas are described following the description of the formulas 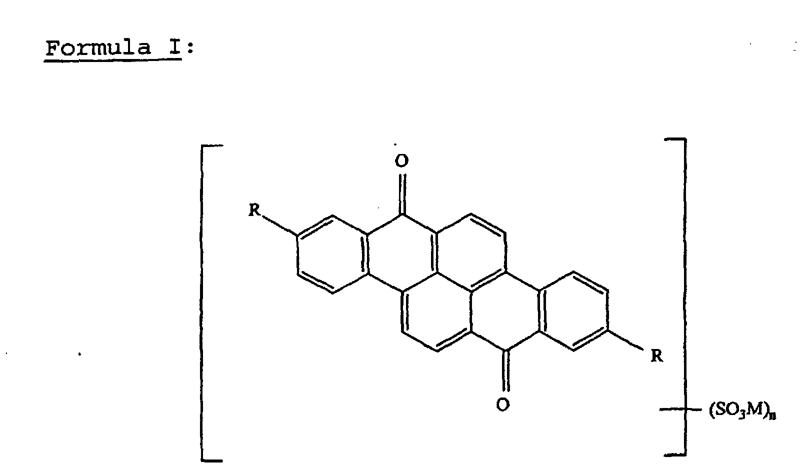 wherein R = Br, NHAr, 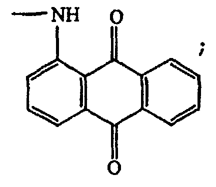 M is a cation; and
n = 2-4. 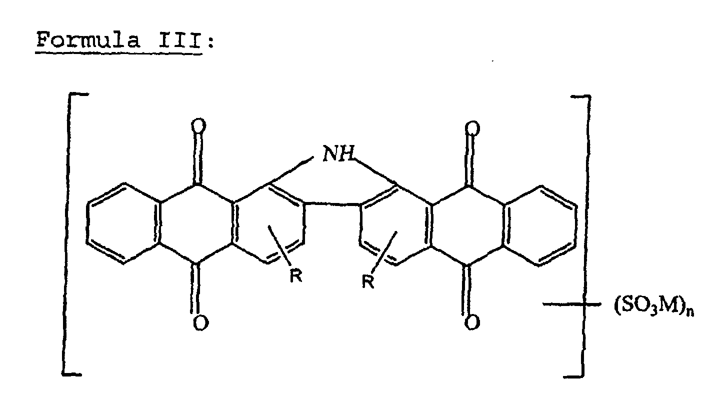 wherein R = H, NHCOPh;
M is a cation; and
n = 2-4. 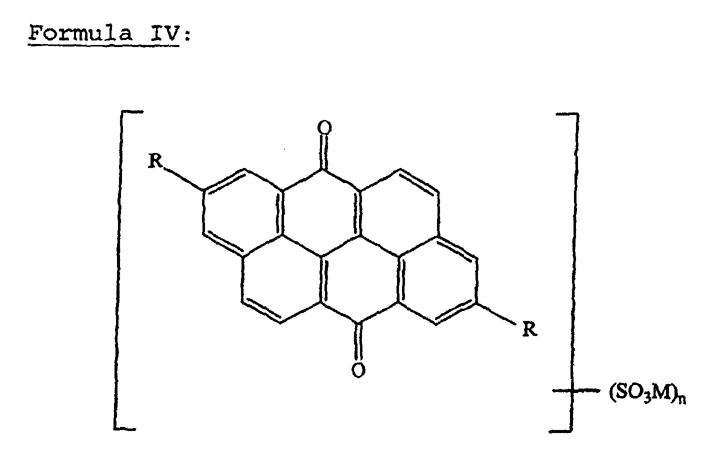 wherein R = H, Br, NHAr, 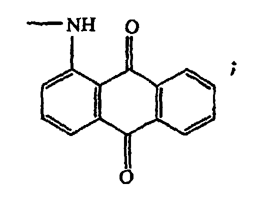 M is a cation; and
n = 2-4. 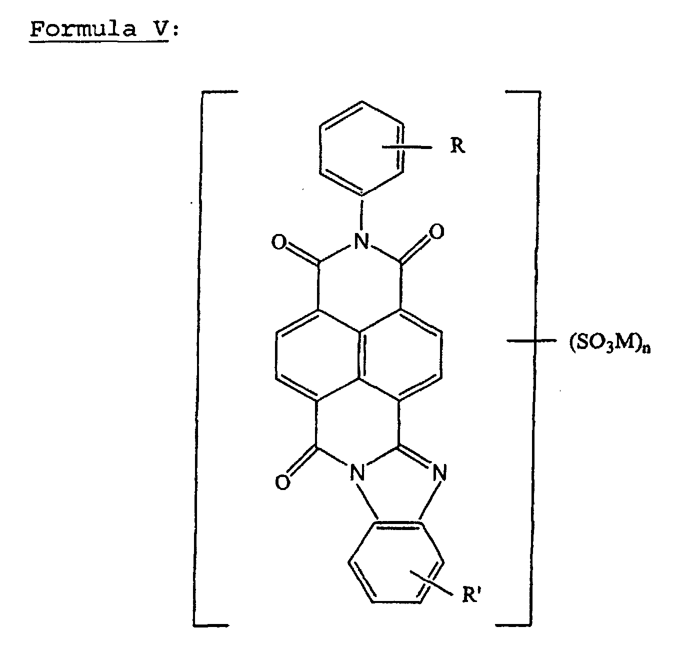 wherein R, R' = H, Hal, Alk, OAlk, ArNH, OPh; M is a cation; and
n = 2-4. 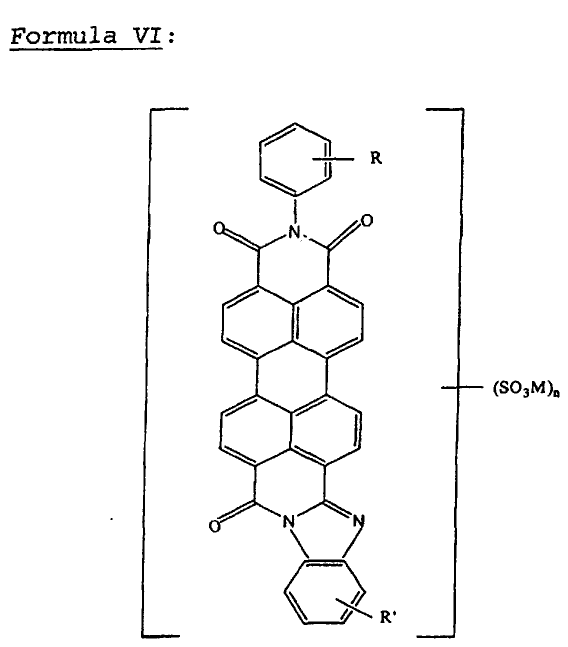 wherein R, R', M, and n are as defined in formula V. 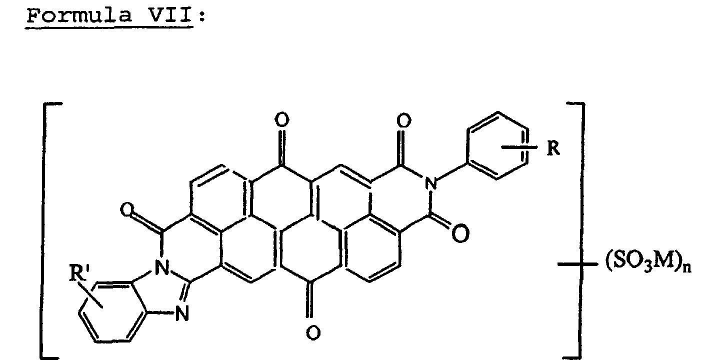 wherein R, R', M, and n are as defined in formula V. 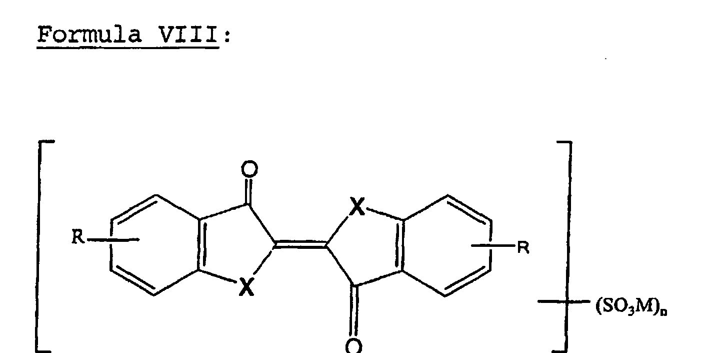 wherein X = S;
R = Hal, AlkO;
M is a cation; and
n = 1-3. 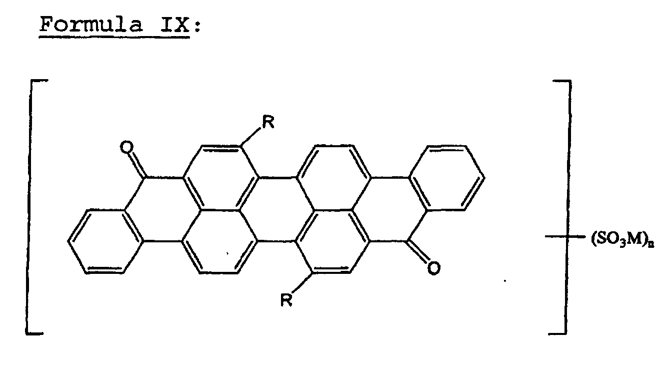 wherein R = OCH₃;
M is a cation; and
n = 2-4. 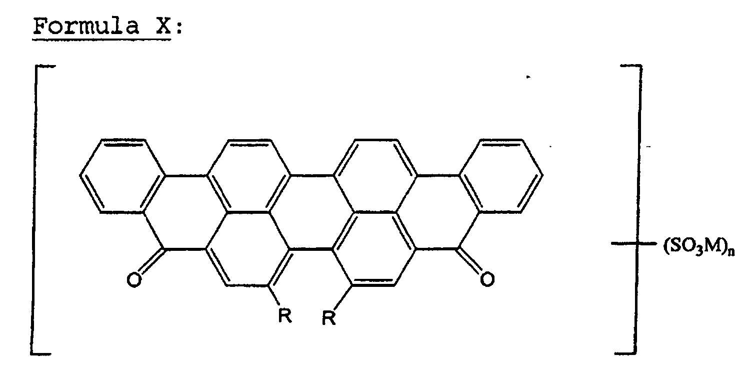 wherein R = OCH₃;
M is a cation; and
n = 2-4.
And wherein "Ar", "Ph", "Hal", "Alk", and "M" in the above Formulae are as described below:
Ar is selected from the group consisting of C₆H₅, Cl-C₆H₄, and C₆H₄SO₃M.
Ph is a phenyl group.
Alk is an alkyl group.
Hal is a halide.
M is a cation.
Alk is preferably an alkyl group with from one to four carbon atoms.
Hal is preferably Cl or Br.
M is preferably selected from the group consisting of H⁺, Li⁺, Na⁺, K⁺, Cs⁺, or NH₄⁺.

This invention is further illustrated by the following specific but non-limiting examples. Temperatures are given in degrees Centigrade and concentrations as weight percent unless otherwise specified. Procedures which are constructively reduced to practice are described in the present tense, and procedures which have been carried out in the laboratory are set forth in the past tense. In the examples, the elemental analysis for each sample was determined twice. All citations in the above specification and the following examples are incorporated herein by reference in their entireties.

### EXAMPLE 1

### Synthesis of a Dye of Formula I

In this example, a dye of formula I was synthesized by various procedures.

### Synthesis of Dye

In this example, a dye of formula I in which R = H, M = H⁺, n = 2, was prepared by the following sulfonation procedure.

A vat yellow 4 (59100 C.I.) dye (5 g) is dissolved in 45-50% oleum (50 ml) to form a reaction mixture. The reaction mixture is heated to 80°C and maintained at this temperature for 7 hours. The reaction mixture is then diluted with 170 ml water, and 40 g sodium chloride is added. The reaction mixture suspension is heated to 80°C, maintained at this temperature for 10 minutes, and filtered in the hot state. The residue is pressed and washed with a 13% solution of sodium chloride until no sulfate anions are detected in the filtrate. The residue is dried, boiled in 30 ml of 16% hydrochloric acid, and filtered to form a final residue. The final residue is washed with 10% hydrochloric acid, then with isopropyl alcohol, and dried.

This sulfonation procedure yielded 5.06 g dye with formula I, n = 2. The elemental analysis calculated for C₂₄H₁₂O₈S₂·4H₂O was S 11.37. The elemental analysis determined was S 11.69, 11.88.

The above sulfonation procedure was repeated using anthanthrone (5.0 g) instead of vat yellow 4 dye and produced 4.85 g dye of formula IV, n = 2. The elemental analysis calculated for C₂₃H₁₁O₈S₂ was S 13.37. The elemental analysis determined was S 13.12, 13.56.

The above sulfonation procedure was repeated using isoviolanthrone (5 g) instead of vat yellow 4 dye and produced 5.38 g dye of formula IX, n = 3. The elemental analysis calculated for C₃₄H₁₆O₁₁S₃·3H₂O was S 12.82. The elemental analysis determined was S 12.58, 13.23.

The above sulfonation procedure was repeated using violanthrone (5 g) instead of vat yellow 4 dye and produced 5.5 g dye of formula X, n = 4. The elemental analysis calculated for C₃₄H₁₆O₁₄S₄·4(H₂O) was S 15.09. The elemental analysis determined was S 14.80, 14.95.

### EXAMPLE 2

### Synthesis of a Dye of Formula I

Synthesis of a dye of formula I, R = H, M=H⁺, n = 3 was performed as described below.

A vat yellow 4 (59100 C.I.) dye (5 g) is dissolved in 45-50% oleum (50 ml). Then mercury sulfate (0.03 g) is added to form a reaction mixture. The reaction mixture is heated to 115°C. After keeping the mixture at 110-120°C for 8 hours, the reaction mixture is diluted with water to a sulfuric acid concentration of 50%, and 25 g sodium chloride is added. The reaction mixture suspension is heated to 80°C and filtered in the hot state. The residue is washed with a 12% solution of sodium chloride, 16% hydrochloric acid, and isopropyl alcohol, and dried to yield 5.4 g dye with formula I, n = 3. The elemental analysis calculated for C₂₄H₁₂O₁₁S₃·3(H₂O) was S 15.36. The elemental analysis determined was S 14.89, 15.03.

The sulfonation procedure described in this example was repeated using vat orange 1 (59105 C.I.) dye (5 g) instead of vat yellow 4 dye and produced a dye of formula I, R = Br, n = 2. The elemental analysis calculated for C₂₄H₁₀Br₂O₈S₂·2(H₂O) was Br 23.32; S 9.33. The elemental analysis determined was Br 23.44, 23.68; S 9.00, 9.12.

The sulfonation procedure described in this example was repeated using 3,9-di(anthraquinonyl-1-amino)dibenzepyrenquinone (5.0 g) instead of vat yellow 4 dye and produced 6.2 g dye of formula I, and produced a dye of formula I, R = anthraquinonyl-1-amino, n = 4. The elemental analysis calculated for C₅₂H₂₈N₂O₁₈S₄·4(H₂O) was N 2.39; S 10.96. The elemental analysis determined was N 2.40, 2.54; S 10.78, 10.81.

The sulfonation procedure described in this example was repeated using vat grey '2S' dye (5 g) instead of vat yellow 4 dye and produced 6.0 g dye of formula IV, R = anthraquinonyl-1-amino, n = 4. The elemental analysis calculated for C₅₀H₂₄N₂O₁₈S₄·4(H₂O) was N 2.46; S 11.23. The elemental analysis determined was N 2.36, 2.61; S 10.98, 11.00.

### EXAMPLE 3

### Synthesis of a Dye of Formula III

A dye of formula III, R = H, n = 2 was prepared as described below.

5 g diphthaloylcarbazole is dissolved in a mixture of 20% oleum (10 ml) and chlorosulfonic acid (20 ml) to form a reaction mixture. The reaction mixture is heated at 85-90°C for 10-12 hours. The reaction mixture is then cooled and diluted with water to a sulfuric acid concentration of 50%. The residue in the reaction mixture is filtered, washed with 16% hydrochloric acid, and dried. The product is dissolved in 150 ml water at pH 6, and 100 ml isopropyl alcohol is added. The precipitate is filtered, washed with a water-isopropanol mixture (1:1 v/v), and dried to yield 5.8 g dye with formula III, R = H, n = 2. The elemental analysis calculated for C₂₈H₁₃NO₁₀S₂·4(H₂O) was S 9.73; N 2.12. The elemental analysis determined was S 9.34, 9.65; N 2.00, 2.35.

The sulfonation procedure described in this example was repeated using 4,5'- dibenzoylaminodiphthaloylcarbazole dye (5 g) instead of diphthaloylcarbazole dye and produced 5.1 g dye of formula III, R = NHCOPh, n = 3. The elemental analysis calculated for C₄₂H₂₃N₃O₁₅S₃·3(H₂O) was S 10.94; N 4.38. The elemental analysis determined was S 10.36, 10.55; N 4.07, 4.26.

The sulfonation procedure described in this example was repeated using 5,5'- dibenzoylaminodiphthaloylcarbazole dye (5 g) instead of diphthaloylcarbazole and produced 5.0 g dye of formula III, R = NHCOPh, n = 4. The elemental analysis calculated for C₄₂H₂₃N₃O₁₈S₃ was S 12.13; N 4.26. The elemental analysis determined was S 11.87, 11.98; N 3.98, 4.11.

The sulfonation procedure described in this example was repeated using 1,4,5,8-naphthalinetetracarboxylic acid (NTCA) hydroxyphenylimide methylbenzimidazole dye (5 g) instead of diphthaloylcarbazole dye and produced 5.3 g dye of formula V, R = OH, R' = CH₃, n = 3. The elemental analysis calculated for C₂₇H₁₅N₃O₁₃S₃·3(H₂O) was N 5.68; S 12.99. The elemental analysis determined was N 5.33, 5.60; S 12.00, 12.23.

The sulfonation procedure described in this example was repeated using NTCA butylphenylimide chlorobenzimidazole dye (5 g) instead of diphthaloylcarbazole dye and produced 4.8 g dye of formula V, R = C₄H₉, R' = Cl, n = 2. The elemental analysis calculated for C₃₀H₂₀ClN₃O₉S₂·2(H₂O) was Cl, 5.06; N 5.99; S 9.12. The elemental analysis determined was Cl, 4.68, 5.12; N 5.33, 5.60; S 8.90, 9.23.

The sulfonation procedure described in this example was repeated using NTCA ethoxyphenylimide methylbenzimidazole dye (5 g) instead of diphthaloylcarbazole dye and produced 5.4 g dye of formula V, R = C₂H₅O, R' = CH₃, n = 3. The elemental analysis calculated for C₂9H₁₉N₃O₁₃S₃·3(H₂O) was N 5.48; S 2.52. The elemental analysis determined was N 5.00, 5.32; S 11.90, 12.45.

The sulfonation procedure described in this example was repeated using NTCA bromophenylimide methylbenzimidazole dye (5 g) instead of diphthaloylcarbazole dye and produced 5.0 g dye of formula V, R = Br, R' = CH₃, n = 2. The elemental analysis calculated for C₂₉H₁₉N₃O₁₃S₃·3(H₂O) was N 5.48; S 12.52. The elemental analysis determined was Br, 10.77, 10.98; N 5.45, 5.71; S 9.56, 9.79.

The sulfonation procedure described in this example was repeated using 3,4,9,10-perylenetetracarboxylic acid (PTCA) hydroxyphenylimide methylbenzimidazole dye (5 g) instead of diphthaloylcarbazole dye and produced 5.5 g dye of formula VI, R = OH, R' = CH₃, n = 3. The elemental analysis calculated for C₃₁H₁₉N₃O₁₃S₃·3(H₂O) was N 5.32; S 12.14. The elemental analysis determined was N 5.54, 5.76; S 11.87, 12.00.

The sulfonation procedure described in this example was repeated using PTCA butylphenylimide chlorobenzimidazole dye (5 g) instead of diphthaloylcarbazole dye and produced 5.2 g dye of formula VI, R = C₄H₉, R' = Cl, n = 2. The elemental analysis calculated for C₃₄H₂₄ClN₃O₉S₂·2(H₂O) was Cl 4.71; N 5.57; S 8.49. The elemental analysis determined was Cl, 4.32, 4.40; N 5.34, 5.39; S 8.90, 9.34.

The sulfonation procedure described in this example was repeated using 3,4,9,10-anthanthronetetracarboxylic acid (AATCA) methoxyphenylimide benzimidazole dye (5 g) instead of diphthaloylcarbazole dye and produced 5.7 g dye of formula VII, R = CH₃O, R' = H, n = 3. The elemental analysis calculated for C₃₉H₁₇N₃O₁₅S₃·3(H₂O) was N 4.75; S 10.85. The elemental analysis determined was N 4.59, 4.76; S 10.45, 10.51.

The sulfonation procedure described in this example was repeated using AATCA methylphenylimide bromobenzimidazole dye (5 g) instead of diphthaloylcarbazole dye and produced 4.5 g dye of formula VII, R = CH₃, R' = Br, n = 2. The elemental analysis calculated for C₃₉H₁₆BrN₃O₁₁S₂·2(H₂O) was Br 9.07; N 4.76; S 7.26. The elemental analysis determined was Br 8.56, 8.70; N 4.33, 4.50; S 7.69, 7.90.

The sulfonation procedure described in this example was repeated using dimethoxyisoviolanthrone dye (5 g) instead of diphthaloylcarbazole dye and produced 5.0 g dye of formula IX, R = OCH₃, n=2. The elemental analysis calculated for C₃₆H₁₈O₁₀S₂·2(H₂O) was S 9.03. The elemental analysis determined was S 9.65, 9.49.

The sulfonation procedure described in this example was repeated using dimethoxyviolanthrone dye (5 g) instead of diphthaloylcarbazole dye and produced 4.9 g dye of formula X, R = OCH₃, n = 2. The elemental analysis calculated for C₃₆H₁₈O₁₀S₂·2H₂O was S 9.03. The elemental analysis determined was S 9.75, 9.60.

### EXAMPLE 4

Synthesis of a dye with formula V, wherein R = R' = H, M=H⁺, n = 2, was performed by the sulfonation procedure described below.

NTCA phenylimide benzimidazole (10 g) is dissolved in 10% oleum (50 ml) to form a reaction mixture. The reaction mixture is heated at 80-85°C for 4 hours. Then, the reaction mixture is cooled and diluted with 100 ml water. The resultant precipitate is filtered, washed with concentrated hydrochloric acid until no sulfate anions are detected in the filtrate, and dried to yield 12.45 g of a dye with formula V, R = R' = H, n = 2. The elemental analysis calculated for C₂₆H₁₃N₃O₉S₂·2(H₂O) was N 7.30; S 11.13. The elemental analysis determined was N 6.98, 7.10; S 11.67, 11.73.

The sulfonation procedure described in this example was repeated using NTCA methoxyphenylimide benzimidazole dye (10 g) instead of NTCA phenylimide benzimidazole dye and produced 13.2 g dye of formula V, R = CH₃O; R' = H, n = 2. The elemental analysis calculated for C₂₇H₁₅N₃O₁₀S₂·2(H₂O) was N 6.55; S 9.98. The elemental analysis determined was N 6.33, 6.40;. S 10.34, 10.50.

The sulfonation procedure described in this example was repeated using NTCA 4-phenylaminophenylimide benzimidazole dye (10 g) instead of NTCA phenylimide benzimidazole dye and produced 15.0 g dye of formula V, R = NHPh, R' = H, n = 4. The elemental analysis calculated for C₃₂H₁₈N₄O₁₅S₄·4(H₂O) was N 6.24; S 14.25. The elemental analysis determined was N 5.89, 6.10; S 13.90, 14.11.

The sulfonation procedure described in this example was repeated using NTCA 4-phenyloxyphenylimide benzimidazole dye (10 g) instead of NTCA phenylimide benzimidazole dye and produced 12.3 g dye of formula V, R = OC₆H₅, R' = CH3, n = 3. The elemental analysis calculated for C₃₃H₁₉N₃O₁₃S₃·3(H₂O) was N 5.15; S 11.78. The elemental analysis determined was N 4.78, 5.10; S 11.45, 11.63.

The sulfonation procedure described in this example was repeated using PTCA phenylimide benzimidazole dye (10 g) instead of NTCA phenylimide benzimidazole dye and produced 11.8 g dye of formula VI, R = R' = H, n = 2. The elemental analysis calculated for C₃₀H₁₇N₃O₉S₂·2(H₂O) was N 6.33; S 9.65. The elemental analysis calculated for N 5.87, 5.90; S 9.99, 10.12.

The sulfonation procedure described in this example was repeated using PTCA methoxyphenylimide benzimidazole dye (10 g) instead of NTCA phenylimide benzimidazole dye and produced 12.0 g dye of formula VI, R = CH₃O, R' = H, n = 2. The elemental analysis calculated for C₃₁H₁₉N₃O₁₀S₂·2(H₂O) was N 5.91; S 9.00. The elemental analysis determined was N 5.34, 5.60; S 9.45, 9.63.

The sulfonation procedure described in this example was repeated using PTCA 4-phenylaminophenylimide benzimidazole dye (10 g) instead of NTCA phenylimide benzimidazole dye and produced 14.5 g dye of formula VI, R = NHPh,, R = NHPh, R' = H, n = 4. The elemental analysis calculated for C₃₆H₂₂N₄O₁₅S₄·4(H₂O) was N 5.89; S 13.47. The elemental analysis determined was N 5.46, 5.66; S 13.00, 13.47.

The sulfonation procedure described in this example was repeated using PTCA ethoxyphenylimide bromobenzimidazole dye (10 g) instead of NTCA phenylimide benzimidazole dye and produced 10.5 g dye of formula VI, R = C₂H₅O, R' = Br, n = 2. The elemental analysis calculated for C₃₂H₂₀BrN₃O₁₀S₂·2(H₂O) was Br, 10.18; N 5.34; S 8.14. The elemental analysis determined was Br, 9.78, 9.90; N 4.98, 5.12; S 8.23, 8.45.

The sulfonation procedure described in this example was repeated using AATCA phenylimide benzimidazole dye (10 g) instead of NTCA phenylimide benzimidazole dye and produced 10.8 dye of formula VII, R = R' = H, n = 2. The elemental analysis calculated for C₃₈H₁₅N₃O₁₁S₂·2(H₂O) was N 5.32; S 8.11. The elemental analysis determined was N 4.87, 4.90; S 8.45, 8.64.

The sulfonation procedure described in this example was repeated using AATCA ethoxyphenylimide chlorobenzimidazole dye (10 g) instead of NTCA phenylimide benzimidazole dye and produced 11.8 dye of formula VII, R = C₂H₅O, R' = Cl, n = 2. The elemental analysis calculated for C₄₀H₁₈ClN₃O₁₂S₂·2(H₂O) was Cl, 4.08; N 4.83; S 7.36. The elemental analysis determined was Cl, 3.67, 3.90; N 4.34, 4.51.

The sulfonation procedure described in this example was repeated using AATCA 4-phenyloxyphenylimide benzimidazole dye (10 g) instead of NTCA phenylimide benzimidazole dye and produced 13.0 g dye of formula VII, R = OPh, R' = CH₃, n = 3. The elemental analysis calculated for C₄₅H₂₁N₃O₁₅S₃·3(H₂O) was N 4.23; S 9.67. The elemental analysis determined was N 3.99, 4.27; S 9.45, 9.81.

### EXAMPLE 5

Synthesis of a dye with formula VIII, X = S, R = CH₃O, M=H⁺ was performed by the sulfonation procedure described below.

Dimethoxythioindigo (10 g) is dissolved in 18-25% oleum (50 ml) to form a reaction mixture. The reaction mixture is stored at room temperature for 15-18 hours until obtaining a water-soluble probe. (By "obtaining a water-soluble probe" is meant that 0.1 ml of the reaction mixture is diluted with 10 ml of water. When the reaction mixture dissolves in the water, a water-soluble probe is obtained.) Then, the reaction mixture is diluted with water to a sulfuric acid concentration of 50%, and 25 g sodium chloride is added. The resultant suspension is heated to 50°C and filtered in the hot state. The residue is washed with a 15% solution of sodium chloride, then with 16% hydrochloric acid until no sulfate anions are detected in the filtrate. The filtrate is then dried. The dry residue is boiled in 100 ml of ethyl alcohol, hot filtered, washed with 20 ml ethyl alcohol, and dried to yield 12.4 g dye with formula VIII, R = CH₃O. The elemental analysis calculated for C₁₈H₁₂O₁₀S₄·4 (H₂O) was S 21.81. The elemental analysis determined was S 21.77, 21.89.

The sulfonation procedure described in this example was repeated using dietoxythioindigo dye (10 g) instead of dimethoxythioindigo dye and produced 12.5 g dye of formula VIII, R = C₂H₅O. The elemental analysis calculated for C₂₀H₁₆O₁₀S₄·4(H₂O) was S 20.82. The elemental analysis determined was S 20.34, 20.42.

The sulfonation procedure described in this example was repeated using dichlorothioindigo dye (10 g) instead of dimethoxythioindigo dye and produced 10.5 g dye of formula V, R = Cl. The elemental analysis calculated for C₁₆H₆Cl₂O₈S₄·2(H₂O) was S 22.86; Cl, 12.64. The elemental analysis determined was S 22.26, 22.44; Cl, 12.09, 12.23.

## Claims

1. A dye having a formula selected from the group consisting Formula I, III, IV, V, VI, VII, VIII, IX, and X;
wherein the Formulae are as defined below: 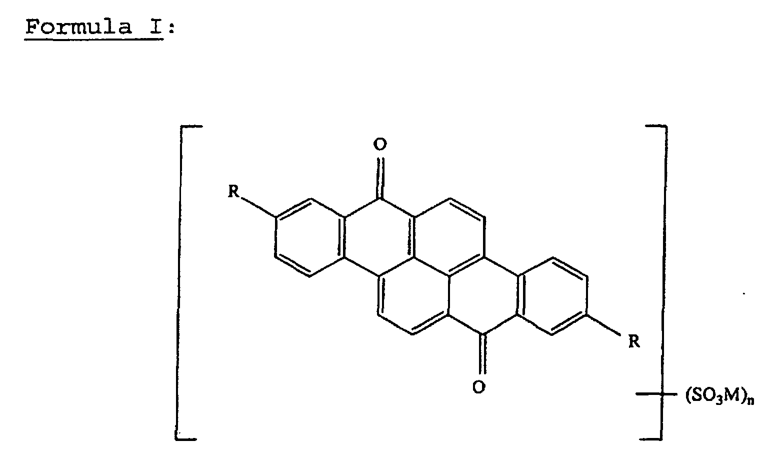 wherein R = Br, NHAr, 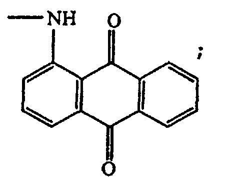 M is a cation; and
n = 2-4. 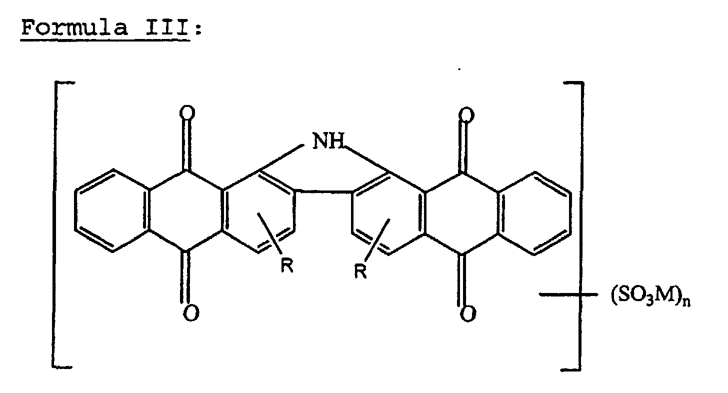 wherein R = H, NHCOPh;
M is a cation; and
n = 2-4. 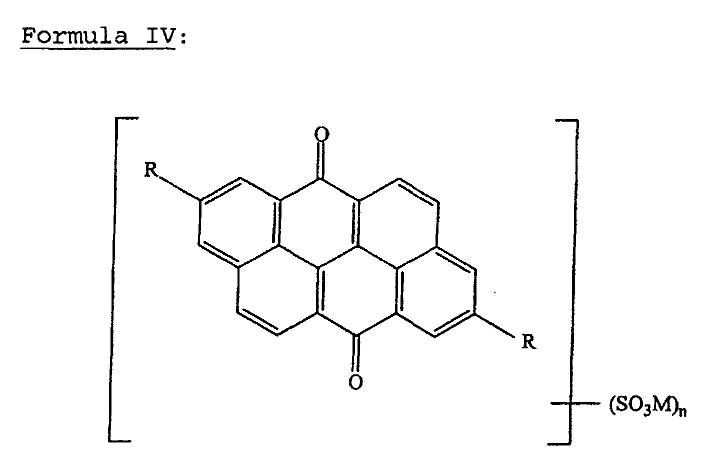 wherein R = H, Br, NHAr, 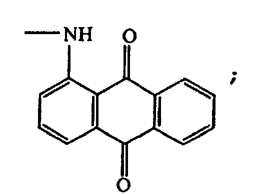 M is a cation; and
n = 2-4. 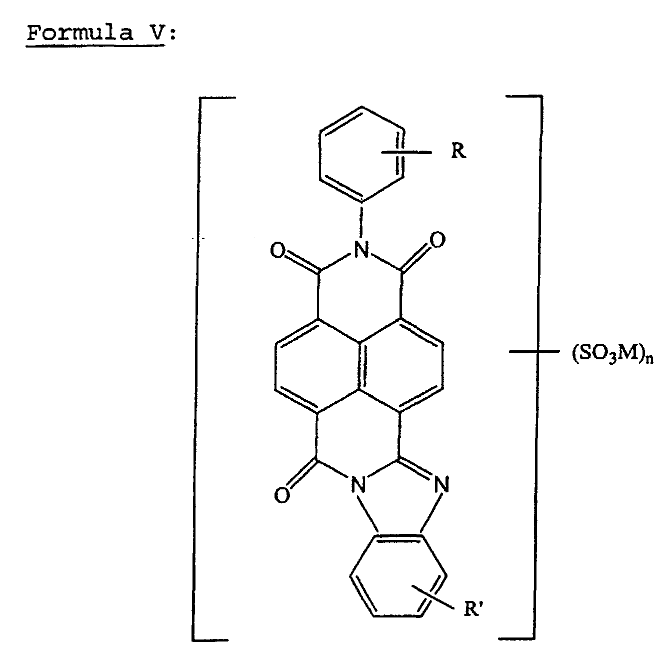 wherein R, R' = H, Hal, Alk, OAlk, ArNH, OPh;
M is a cation; and
n = 2-4. 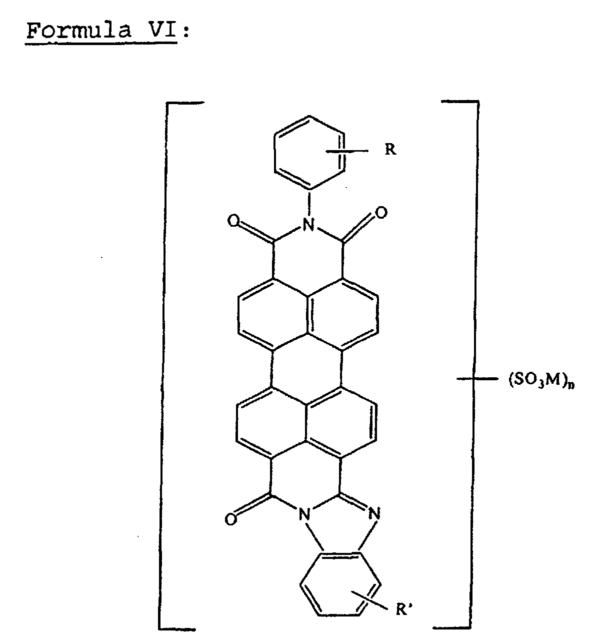 wherein R, R', M, and n are as defined in formula V. 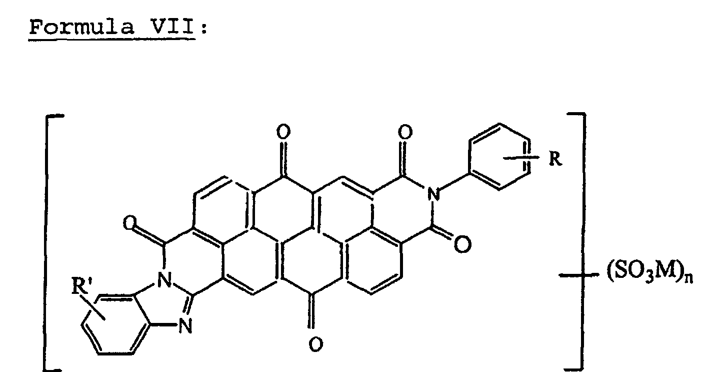 wherein R, R', M, and n are as defined in formula V. 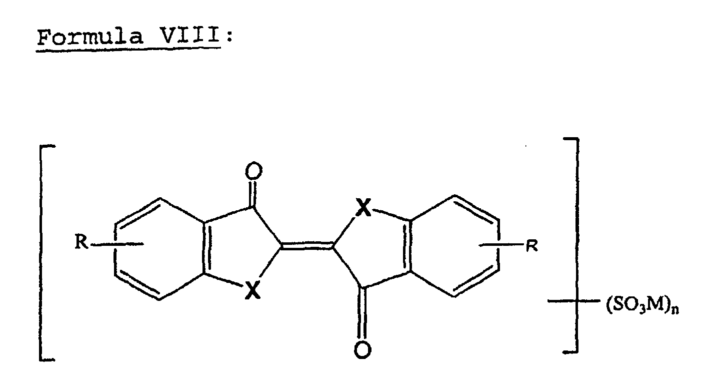 wherein X = S;
R = Hal, AlkO;
M is a cation; and
n = 1-3. 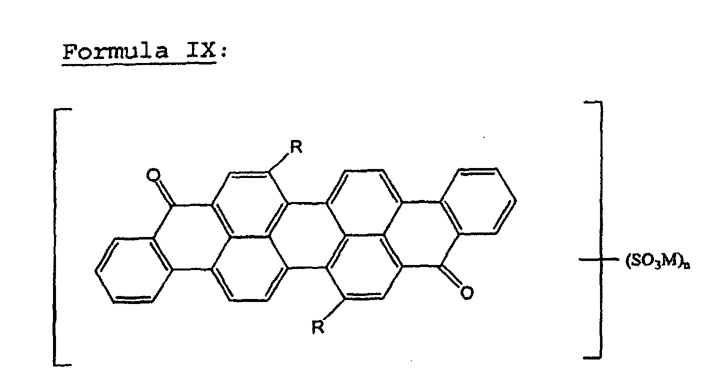 wherein R = OCH₃;
M is a cation; and
n = 2-4. 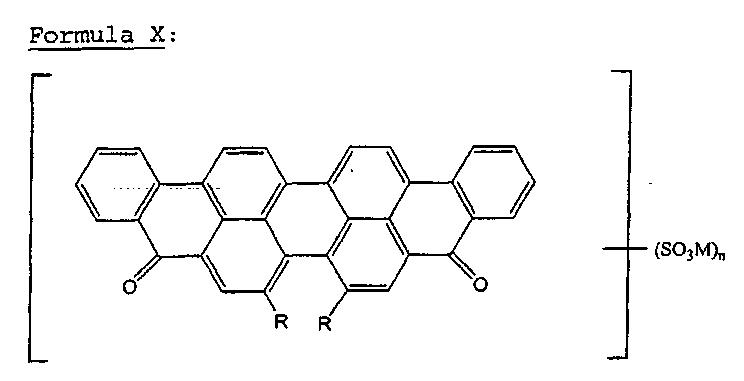 wherein R = OCH₃;
M is a cation; and
n = 2-4.
And wherein "Ar", "Ph", "Hal", "Alk", and "M" in the above Formulae are as described below:
Ar is selected from the group consisting of C₆H₅, Cl-C₆H₄, and C₆H₄SO₃M.
Ph is a phenyl group.
Alk is an alkyl group.
Hal is a halide.
M is a cation.

2. A dye according to claim 1, wherein Alk is an alkyl group with from one to four carbon atoms.

3. A dye according to claim 1 or claim 2, wherein Hal is Cl or Br.

4. A dye according to any of claims 1 to 3, wherein M is selected from the group consisting of H⁺, Li⁺, Na⁺, K⁺, Cs⁺, or NH₄⁺.

## Revendications

1. Colorant présentant une formule choisie dans le groupe constitué des formules I, III, IV, V, VI, VII, VIII, IX et X;
dans lequel les formules sont telles que définies ci-dessous: 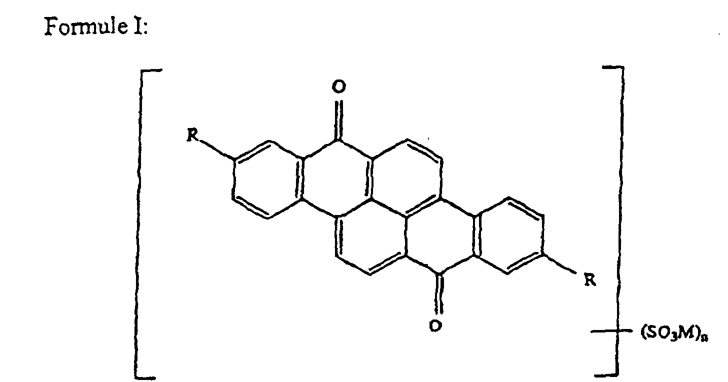 dans laquelle R = Br, NHAr, 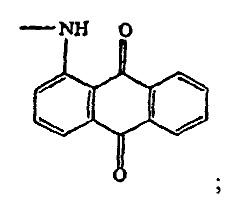 M est un cation; et
n = 2-4, 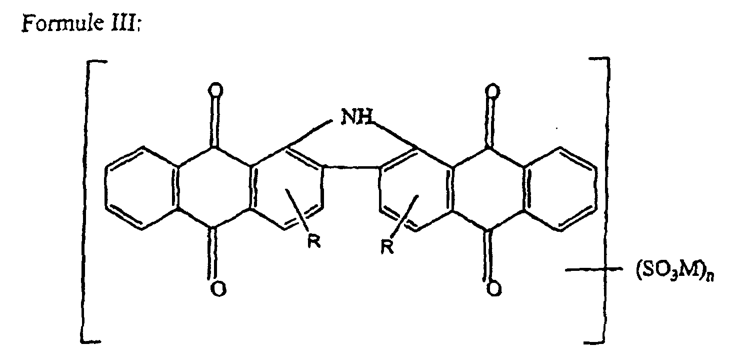 dans laquelle R = H, NHCOPh;
M est un cation; et
n = 2-4. 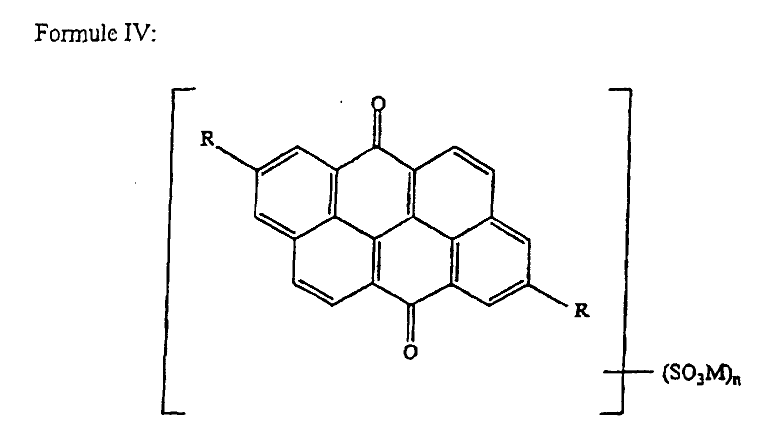 dans laquelle R = Br, NHAr, 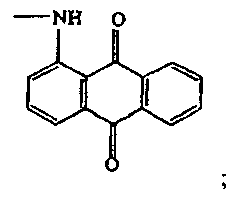 M est un cation; et
n = 2-4. 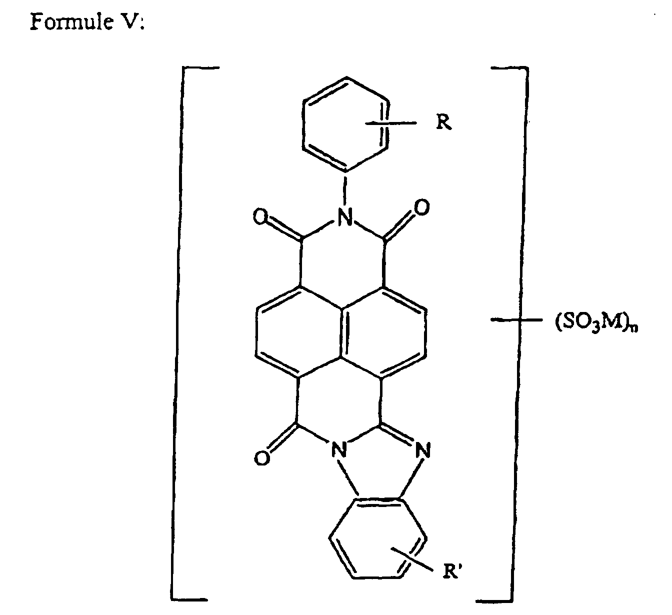 dans laquelle R, R' = H, Hal, Alk, OAlk, ArNH, OPh;
M est un cation; et
n = 2-4. 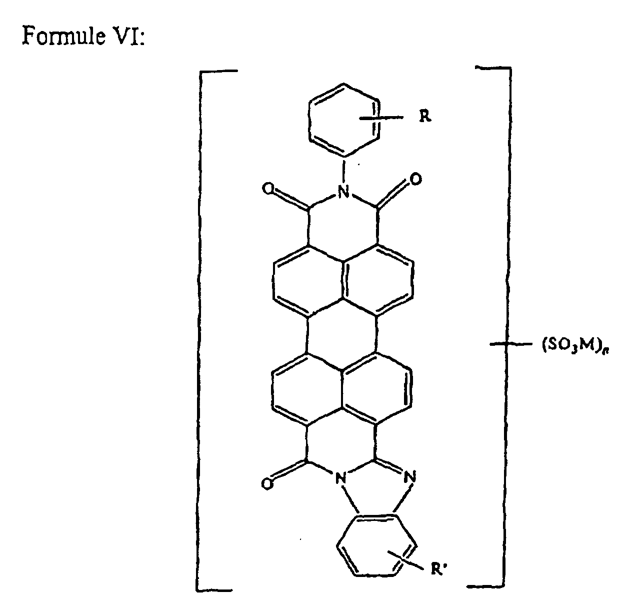 dans laquelle R, R', M et n sont comme il est défini dans la Formule V. 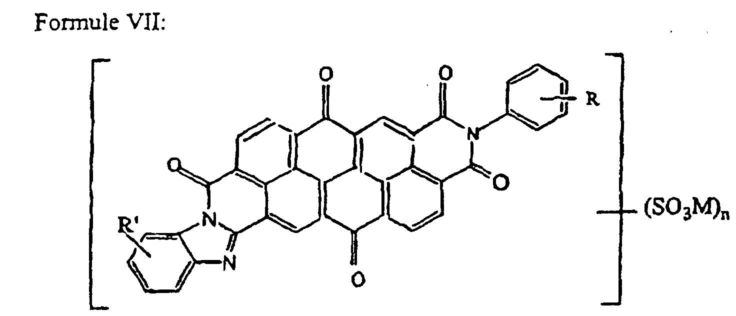 dans laquelle R, R', M et n sont comme il est défini dans la Formule V. 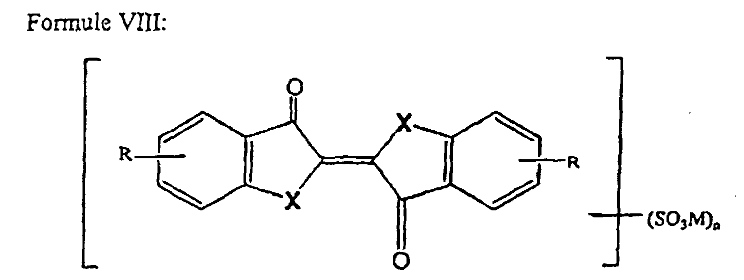 dans laquelle X = S;
R = Hal, AlkO;
M est un cation; et
n = 1-3. 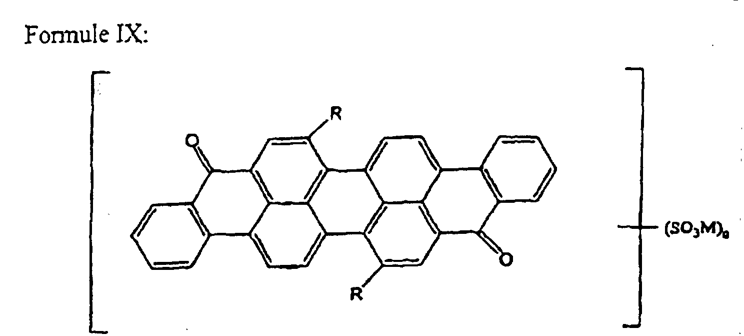 dans laquelle R = OCH₃;
M est un cation; et
n = 2-4. 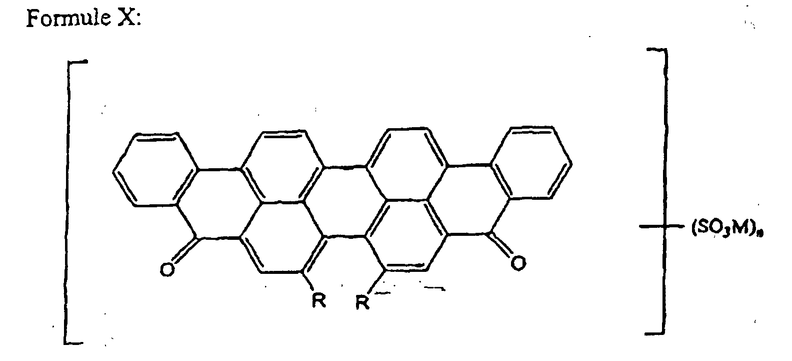 dans laquelle R = OCH₃;
M est un cation; et
n = 2-4.
Et où "Ar", "Ph", "Hal", "Alk" et "M" dans les formules ci-dessus sont comme il est décrit ci-dessous:
Ar est choisi dans le groupe constitué de C₆H₅, Cl-C₆H₄ et C₆H₄SO₃M,
Ph est un groupe phényle,
Alk est un groupe alkyle,
Hal est un halogénure,
M est un cation.

2. Colorant suivant la revendication 1, dans lequel Alk est un groupe alkyle avec de un à quatre atomes de carbone.

3. Colorant suivant la revendication 1 ou la revendication 2, dans lequel Hal est Cl ou Br.

4. Colorant suivant l'une quelconque des revendications 1 à 3, dans lequel M est choisi dans le groupe constitué de H⁺, Li⁺, Na⁺, K⁺, Cs⁺ ou NH₄⁺.

## Patentansprüche

1. Farbstoff mit einer Formel, die aus der Gruppe ausgewählt ist, die aus den Formeln I, III, IV, V, VI, VII, VIII, IX und X besteht;
wobei die Formeln durch die nachstehenden Definitionen gegeben sind: 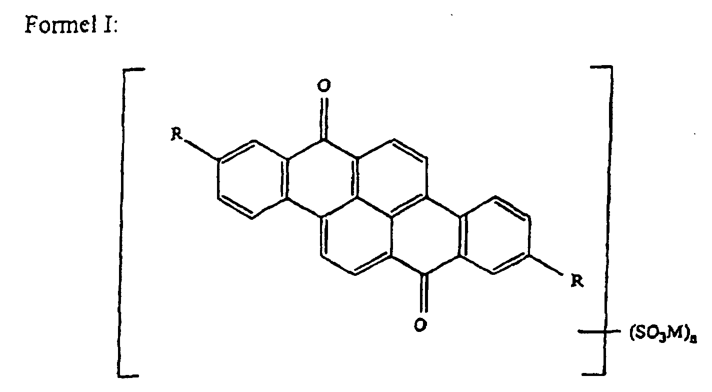 wobei R = BR, NHAr, 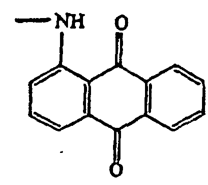 ist;
M ein Kation ist; und
n = 2-4 ist. 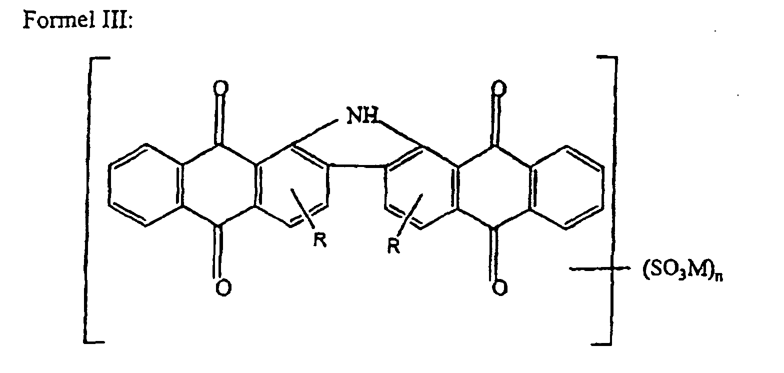 wobei R = H, NHCOPh ist;
M ein Kation ist; und
n = 2-4 ist. 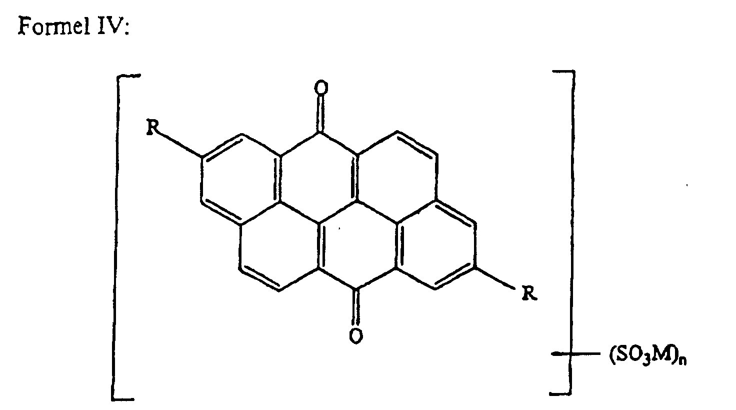 wobei R = H, Br, NHAr, 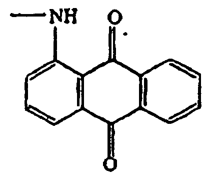 ist;
M ein Kation ist; und
n = 2-4 ist. 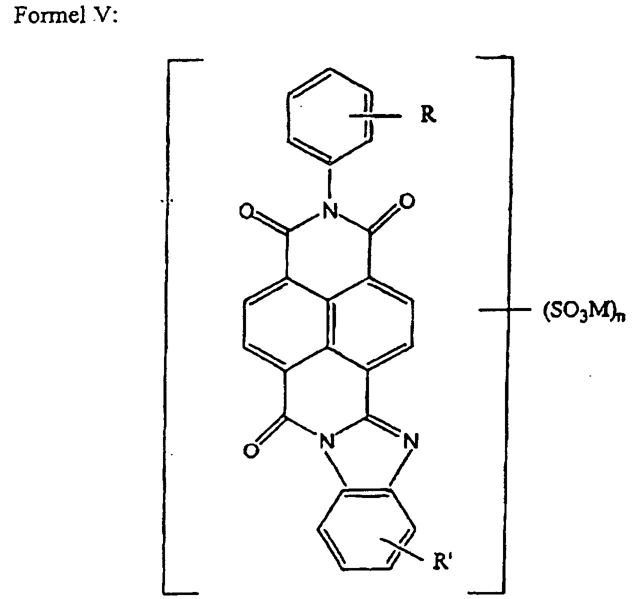 wobei R, R' = H, Hal, Alk, OAlk, ArNH, OPh sind;
M ein Kation ist; und
n = 2-4 ist. 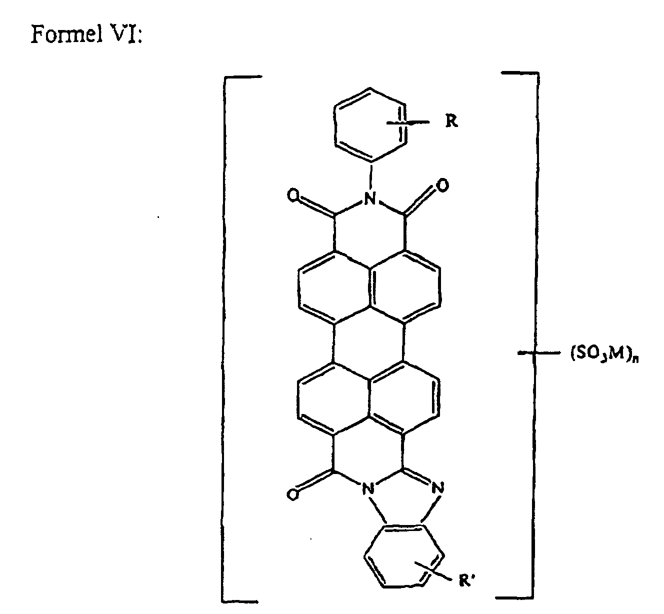 wobei R, R', M und n den Definitionen in Formel V entsprechen. 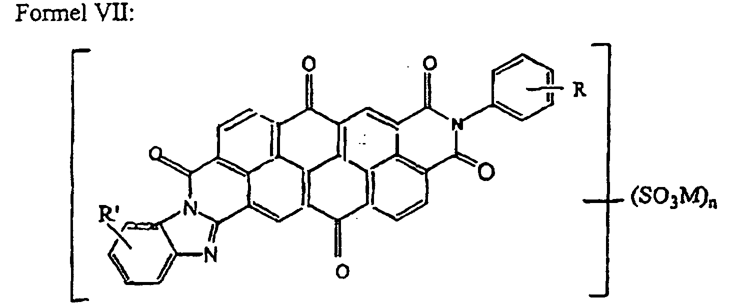 wobei R, R', M und n den Definitionen in Formel V entsprechen. 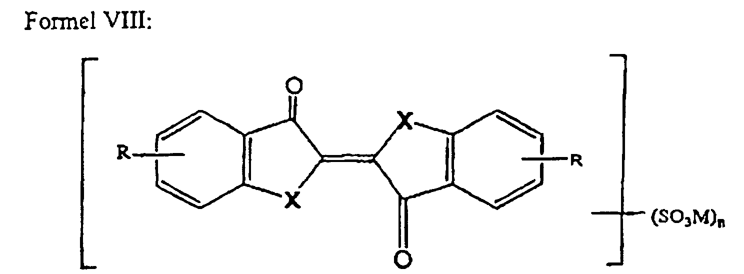 wobei X = S ist;
R = Hal, AlkO ist;
M ein Kation ist; und
n = 1 - 3 ist. 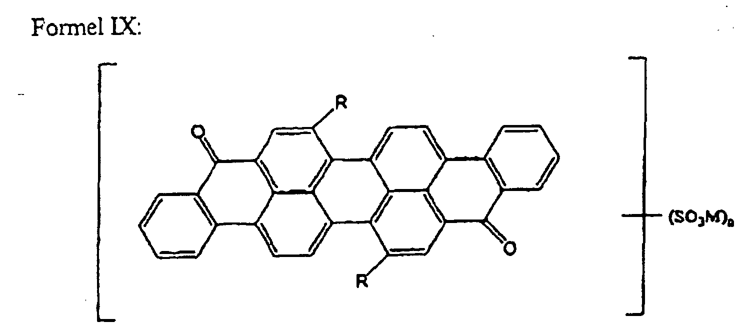 wobei R = OCH₃ ist;
M ein Kation ist; und
n = 2-4 ist. 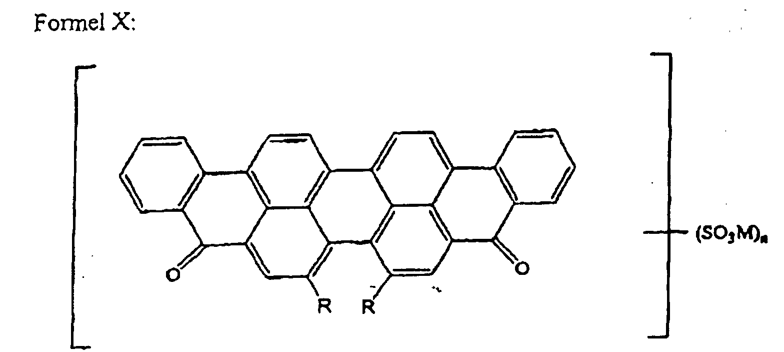 wobei R = OCH₃ ist;
M ein Kation ist; und
n = 2-4 ist;
und wobei "Ar", "Ph", "Hal", "Alk" und "M" in den obigen Formeln sich wie folgt beschreiben lassen:
Ar ist aus der Gruppe ausgewählt, die aus C₆H₅, ClC₆H₄ und C₆H₄SO₃M besteht.
Ph ist eine Phenylgruppe.
Alk ist eine Alkylgruppe.
Hal ist ein Halogenid.
M ist ein Kation.

2. Farbstoff nach Anspruch 1, wobei Alk eine Alkylgruppe mit einem bis vier Kohlenstoffatomen ist.

3. Farbstoff nach Anspruch 1 oder Anspruch 2, wobei Hal Cl oder Br ist.

4. Farbsroff nach einem der Ansprüche 1 bis 3, wobei M aus der Gruppe ausgewählt ist, die aus H⁺, Li⁺, Na⁺, K⁺, Cs⁺ oder NH₄⁺ besteht.
